Europäisches Patentamt

European Patent Office

Office européen des brevets

(19)

(11) Publication number: **0 060 659**
**A1**

(12) # EUROPEAN PATENT APPLICATION

(21) Application number: **82301132.5**

(22) Date of filing: **05.03.82**

(51) Int. Cl.³: **C 07 C 47/127,** C 07 C **149/247,** A 61 K **31/195,** A 61 K **31/11**

(30) Priority: **05.03.81 US 240992**

(43) Date of publication of application: **22.09.82 Bulletin 82/38**

(84) Designated Contracting States: **AT BE CH DE FR GB IT LI LU NL SE**

(71) Applicant: **NATIONAL FOUNDATION FOR CANCER RESEARCH, INC., 7316, Wisconsin Avenue Suite 851, Washington D.C. 20014 (US)**

(72) Inventor: **Gyorgyi, Albert Szent, 7, Winds, Woods Hole Massachusetts (US)**

(74) Representative: **Tubby, David George et al, MARKS & CLERK 57-60 Lincoln's Inn Fields, London WC2A 3LS (GB)**

(54) **Pharmaceutical composition for tumour treatment and process for preparing it.**

(57) An adduct of glyoxal and cysteine containing free radicals is a valuable vehicle for transporting glyoxal (known to have anti-tumour activity) to the tumour site, without interference from the body chemistry. The adduct may be prepared by reacting glyoxal and cysteine in solution and exposing the reaction product to oxygen, preferably by carrying out the reaction under vigorous oxygenation. The adduct may be formulated with conventional pharmaceutical carriers and diluents to prepare a pharmaceutical composition for parenteral or oral use.

EP 0 060 659 A1

ACTORUM AG

PHARMACEUTICAL  COMPOSITION FOR TUMOUR TREATMENT AND PROCESS FOR PREPARING IT

The present invention relates to a pharmaceutical composition comprising an adduct of glyoxal with cysteine, which is useful in the treatment of tumours, and also relates to a process for preparing this adduct.

For many years, scientists have investigated the role of aldehydes in biological systems.  Much of this interest was spurred by the hypothesis of A. Szent-Györgyi ["Electronic Biology and Cancer", M. Dekker Inc., New York (1978)] that these substances, and particularly methylglyoxal and glyoxal, have a regulatory influence on the electronic state of proteins and consequently on cell division. It has been further demonstrated that lipid peroxidation, which leads to the natural production of considerable amounts of aldehydes, is a basic mechanism in cell pathology. Briefly stated, the theory postulates that cancer is a disturbance of the electronic configuration of protein in cancer cells. Aldehydes, such as glyoxal and methylglyoxal, reestablish the proper electronic configuration and cause cancer cells to revert from the abnormal state of proliferation to the normal "resting condition". The mechanism whereby aldehydes achieve this is complex and is not important to the present invention.

There is a second theory which may also explain the anti-tumour activity of certain aldehydes. It has been reported, for example, that 4-hydroxypentenal is only slightly toxic to cells of normal tissues but is highly toxic to tumour cells. This substance is a specific sulphhydryl inhibitor, which blocks DNA, RNA and protein synthesis, as well as glycolysis and respiration in tumour cells [Tillian et al., European Journal of Cancer, 12, 989 (1976)].

Despite the toxicity of aldehydes to tumour cells, a number of practical difficulties prevent their widespread therapeutic use. First, in order to achieve a significant effect, relatively high doses are required but, because of the general toxicity of these compounds even to healthy organisms, such high doses cannot normally be tolerated. Second, methylglyoxal and related compounds are extremely labile in vivo because of the action of a glyoxalase enzyme system which converts them to D-lactic acid in the presence of reduced glutathione and, as a result, an in vivo test of methylglyoxal for its effect on cell division would probably prove negative because of the action of. glyoxalase, which is found throughout all animal tissue. Third, if free aldehydes are administered by intravenous injection, they may immediately bind to serum proteins and to the glutathione present in large amounts in erythrocytes, and thus display no activity.

Accordingly, it has been proposed that, in order to be effective, such aldehydes must be transported to the tumour in the form of a dissociable, less toxic adduct, which will protect the aldehyde from the action of glyoxalase.

One such class of adducts is described in United States patent specification No. 4,238,500 and comprises the products of a reaction between a dialdehyde, α-ketoaldehyde or diketone and an acyloin or enediol compound, such as ascorbic acid. These adducts, and particularly the disclosed methylglyoxal-ascorbic acid adduct, have been shown to be active against various tumours in humans and other animals. It is thought that the effectiveness of these adducts is because they are transported to cancer cells, where they are dissociated to release the aldehyde, which subsequently reacts with proteins of the tumour, resulting in the arrest of cell proliferation.

Adducts of various $\alpha,\beta$-unsaturated aldehydes with cysteine have also been described. For example, the reaction of cysteine with acrolein or with crotonaldehyde can give rise to both mono-adducts and di-adducts [Esterbauer et al., Tetrahedron, 32, 285 (1976)] and the reaction of 4-hydroxypentenal with cysteine has also been shown to produce both mono-adducts and di-adducts. All adducts were found to be in solution in equilibrium with cysteine and the parent aldehydes and the reaction with cysteine has been found to result in a drastic reduction in the toxicity of the aldehyde. Mono-adducts of cysteine with 4-hydroxypentenal and di-adducts of cysteine with crotonaldehyde administered intraperitoneally prevented or inhibited the growth of Ehrlich ascites tumours in mice [Tillian et al., European Journal of Cancer, 12, 989 (1976)].

Schmolka et al. [J. Org. Chem., 22, 943 (1957)] have described adducts of cysteine with various saturated aldehydes. This reaction produces 2-substituted thiazolidine-4-carboxylic acids: for example, the reaction of propionaldehyde with cysteine produces 2-ethylthiazolidine-4-carboxylic acid, but no anti-tumour activity has been reported for these 2-substituted thiazolidine-4-carboxylic acids. An adduct of methylglyoxal with N-acetyl-L-cysteine has also been reported, although the structure has not been elucidated, see Conroy "Cancerostatic activity of methylglyoxal and related substances in tumour-bearing mice" ["Submolecular Biology and Cancer" (edited by Wolstenhome, Fitzsimons and Whalen), Excerpta Medica, New York, pages 271-298 (1979)]. This adduct was four times less toxic than was methylglyoxal alone (on a dose-equivalent basis) but was marginally less effective against Ehrlich ascites carcinoma than was methylglyoxal. There was no significant difference between the patterns obtained with the mono- and di- adducts.

We have now discovered a novel adduct of glyoxal with cysteine which permits the glyoxal to be transported to the site of the tumour to exert its cytostatic effect.

Thus, the present invention provides, in its broadest aspect, an adduct of glyoxal with cysteine containing free radicals.

The invention also provides a process for preparing a glyoxal-cysteine adduct containing free radicals, which process comprises reacting glyoxal and cysteine in solution and exposing the reaction product to oxygen.

The precise structure of the free radical - containing adduct of the invention has not yet been elucidated. It is believed that the adduct can be a 1:2 glyoxal:cysteine adduct or a 1:1 glyoxal: cysteine adduct and, of course, in the normal course of events

- 6 -

0060659

a mixture of these adducts may be formed, the precise proportions of each of the adducts in the mixture depending upon the proportions of glyoxal and cysteine used in the reaction. The formation of a 1:1 or 1:2 adduct is also influenced by the pH of the reaction system. Thus, at acid pH values, the 1:1 adduct tends to be formed whereas the 1:2 adduct is preferentially formed at approximately neutral pH. The pH of the reaction mixture is preferably not too acid and, accordingly, a small quantity of an alkali (such as sodium carbonate) may be added to the reaction mixture to raise the pH value to the appropriate level for the adduct which it is desired to prepare. For example, in order to prepare a 1:1 adduct, we prefer to operate at a pH value within the range from 3 to 6.5, more preferably from 5 to 6, e.g. about 5.5. To achieve neutrality, more alkali may be added to give a substantially neutral pH value, e.g. from 6.5 to 7.5, preferably about 7.

The reaction is preferably effected in aqueous solution and preferably takes place in the presence of oxygen, so that the glyoxal-cysteine adduct is exposed to oxygen immediately that it is formed, thereby giving rise to the free radical-containing adduct.

In a preferred embodiment of the process of the invention, an aqueous solution of cysteine and an aqueous solution of glyoxal are mixed and allowed to react; the concentrations of these solutions are not critical to the present invention and will be chosen having regard to solubility and convenience. However, by way of example, the concentrations may be approximately 0.1 molar for each solution. The amounts of glyoxal and cysteine reacted may also vary, depending upon the nature of the adduct which it is desired to prepare. However, in general, we prefer to employ approximately equimolar amounts of cysteine and glyoxal and determine the nature of the adduct (whether it is a 1:1 or 1:2 adduct) by adjustment of the pH. Prior to mixing, we prefer that each solution should be saturated with oxygen, in order to facilitate formation of the free radical-containing adduct; however, other means of introducing oxygen into the reaction mixture may be chosen, if desired. In order to produce a 1:2 adduct, the pH value of the mixture is then adjusted to about 5.5, e.g. with sodium carbonate, and oxygen is continuously bubbled through the mixture at room temperature. If the 1:1 adduct is desired, additional sodium carbonate or other alkali is employed to give an approximately neutral pH value.

The reaction mixtures described above give a paramagnetic solution, which exhibits a strong electron spin resonance signal, indicating the presence of free radicals. Although these free radicals have not been identified with certainty, it is believed that they are radicals comprising adducts of glyoxal and cysteine.

The adducts of the present invention exhibit a wide range of activity against various experimental tumour systems, e.g. P-388 lymphatic leukemia, Lewis lung carcinoma and L-1210 leukemia, combined with a low toxicity. The adducts may be administered either alone or in combination with other anti-tumour agents. They are preferably administered parenterally in the form of a pharmaceutical composition comprising a mixture of the active agent with a suitable pharmaceutical carrier or diluent, but they may also be administered orally. The pharmaceutical composition may be in any conventional form suitable for injection or oral administration, for example in the form of an ampoule, capsule, tablet, powder or granule.

The amount of adduct administered will vary depending upon the particular adduct used, the particular formulation, the mode and route of administration and many other factors peculiar to the patient concerned (e.g. age, body weight, sex, diet, time of administration, route of administration, rate of metabolism or excretion, drug combination, sensitivities and severity or condition of the disease) and, accordingly, the particular dose may vary widely and will be determined by the physician in the course of treatment, based on conventional tests. However, a suitable dosage for adult humans is from 0.3 to 5 mg/kg body weight daily; this may be administered as a single dose or in divided doses.

The process of the invention is illustrated by the following Example 1, whilst the activities of the adducts of the invention are illustrated in the following Examples 2-5.


Example 1


5.8 g of an oxygen-saturated, 40% w/w solution of glyoxal in water were mixed with 100 ml of an oxygen-saturated aqueous solution containing 5 g of cysteine. Sufficient sodium carbonate was added to the solution to adjust the pH value to about neutrality, thus preparing the 1:1 adduct. In a separate experiment, sufficient sodium carbonate was added to adjust the pH value to about 5.5, giving the 1:2 adduct.


In each case, oxygen was bubbled through the mixture at room temperature for 50 minutes. At the end of this time, a brilliantly coloured yellow-red precipitate formed and was separated. The precipitate was amorphous and dissociated upon heating. In each case, the product was believed to be a mixture containing the 1:1 and 1:2 adducts, the predominant component depending upon the pH at which the reaction was conducted. Infrared spectra were amorphous and uninformative. Nuclear magnetic resonance and electron spin resonance spectra were consistent with a large number of structural possibilities and the final structure of the product has not been determined precisely.

## Example 2

30 adult female white Swiss mice were inoculated behind the right foreleg on the ventral side with about $5 \times 10^6$ Ehrlich ascites cells in 0.2 ml of physiological saline. The mice were randomized and divided into three groups, each group containing 10 animals. All animals received water to drink during days 1 and 2. On day 3, the water of one group (Group B) was replaced by a 0.05 molar solution of glyoxal whilst the water of another group (Group C) was replaced by a 0.05 molar solution of a glyoxal-cysteine adduct prepared essentially as described in Example 1. The third, or control, group (Group A) continued to receive water. All groups could drink ad libidum.

After 7 days, all animals appeared healthy and exhibited no toxic symptoms or loss of weight. No significant decrease was seen on visual inspection in size or form of the tumour in Groups A and B. However, the tumours of Group C (i.e. the group receiving the adduct in their drinking water) appeared about one half the size of the tumours of the control group. No weight loss was observed.

All animals were narcotized and their tumours excised and cleaned from the surrounding tissues. The weight of the excised tissues is given in Table 1.

Table 1

| Group A | Group B | Group C |
|---|---|---|
| 460 | 190 | 270* |
| 1600 | 90* | 120* |
| 1300 | 1270 | 300 |
| 1770 | 330 | 0 (No tumour) |
| 1470 | 1220 | 0 (No tumour) |
| 1570 | 1700 | 250 |
| 2080 | 2740 | 700 |
| 1150· | 980 | 800 |
| 250* | 800 | 1600 |
| 360* | 530 | 470 |

*Indicates animal developed a slightly ascitic tumour.

In the cases where the tumour which developed was slightly ascitic, the fluid was not weighed and this is believed to account for the rather lower weight of these tumours. The average weight of the tumours of Group A was 1200, that of Group B was 985 and that of Group C was 450, corresponding to a 63% inhibition of Group C over the control. The inhibition of Group B was about 18%, which is consistent with the known relatively modest activity of glyoxal itself.

## Examples 3 to 5

The adducts of the present invention were also tested against several transplantable mouse tumours, the essential experimental details for each Example being summarised below. The methodology used generally followed the protocols of the National Cancer Institute for lymphatic leukemias P-388 and L-1210 and for Lewis lung carcinoma [Cancer Chemotherapy Reports, 50, 79 (1966) and Cancer Chemotherapy Reports Part 3, 3, 1 (1972)]. The term "di-adduct" refers to the glyoxal-cysteine 1:2 reaction product and the term "mono-adduct" refers to the glyoxal-cysteine 1:1 reaction product.

### Example 3

Effect of adduct on P-388 mouse leukemia

| Compounds | Dose mg/kg/day |
|-----------|----------------|
| Di-adduct | 32 |
| Di-adduct | 16 |
| Mono-adduct | 32 |
| Mono-adduct | 16 |
| None | Saline |

Tumour inoculum: $10^6$ ascitic cells implanted intraperitoneally

Host: $CDF_1$ male mice with P-388.

Treatment: Once daily for 9 days starting Day 1.

## Example 4

Effect of adducts on Lewis Lung Carcinoma

| Compound | Dose mg/kg/day |
|----------|----------------|
| Di-adduct | 2 |
| Di-adduct | 1 |
| Di-adduct | 0.5 |
| Mono-adduct | 8 |
| Mono-adduct | 4 |
| Mono-adduct | 2 |
| Mono-adduct | 1 |
| Mono-adduct | 0.5 |
| None | Saline |

Tumour inoculum: $1 \times 10^6$ cells from minced tumour brei implanted intraperitoneally.

Host: $BDF_1$ male mice.

Treatment: Once daily for 11 days starting Day 1.

Example 5

Effect of adducts on L-1210 leukemia

| Compound | Dose mg/kg/day |
|----------|----------------|
| Di-adduct | 32 |
| Di-adduct | 16 |
| Di-adduct | 8 |
| Di-adduct | 4 |
| Di-adduct | 2 |
| Di-adduct | 1 |
| Di-adduct | 0.5 |
| Mono-adduct | 32 |
| Mono-adduct | 16 |
| Mono-adduct | 8 |
| Mono-adduct | 4 |
| Mono-adduct | 2 |
| Mono-adduct | 1 |
| Mono-adduct | 0.5 |
| None | Saline |

Tumour inoculum:  $10^6$ ascitic cells implanted intraperitoneally.

Host:  $BDF_1$ male mice.

Treatment:  Once daily for 9 days starting day 1.

## SUMMARY OF RESULTS

Example 3: The di-adduct and mono-adduct, tested on P-388 lymphatic leukemia were both found highly active.

Example 4: The mono-adduct and di-adduct were tested on Lewis lung carcinoma. The mono-adduct gave excellent anti-tumour effects and resulted in long term survival at the doses employed. The di-adduct was effective in increasing median survival time.

Example 5: The mono-adduct and di-adduct, tested on L-1210 Leukemia, both gave comparable results on the daily dose schedule employed, resulting in statistically significant increases in median survival times and long term survival rates.

CLAIMS

1.      An adduct of glyoxal with cysteine, containing free radicals.

2.      An adduct according to claim 1, wherein the molar ratio of glyoxal to cysteine is about 1:1.

3.      An adduct according to claim 1, wherein the molar ratio of glyoxal to cysteine is about 1:2.

4.      A process for preparing a glyoxal-cysteine adduct containing free radicals, which process comprises reacting glyoxal and cysteine in solution and exposing the reaction product to oxygen.

5.      A process as claimed in claim 4, wherein the solution is an aqueous solution.

6.      A process as claimed in claim 4 or claim 5, wherein the reaction mixture is exposed to oxygen throughout the reaction period.

7.      A process as claimed in claim 6, wherein the reaction mixture is exposed to oxygen by bubbling oxygen through it.

8.     A process as claimed in any one of claims 4 to 7, wherein the reaction is effected by mixing an aqueous solution of cysteine with an aqueous solution of glyoxal.

9.     A process as claimed in claim 8, wherein both said aqueous solutions are saturated with oxygen.

10.     A process as claimed in any one of claims 4 to 9, wherein the reaction is effected at a pH of from 6.5 to 7.5, to prepare a 1:1 glyoxal:cysteine adduct.

11.     A process as claimed in any one of claims 4 to 9, wherein the reaction is effected at a pH of from 3 to 6.5, to prepare a 1:2 glyoxal:cysteine adduct.

12.     A process as claimed in claim 11, wherein said pH is about 5.5.

13.     A pharmaceutical composition comprising an adduct as claimed in any one of claims 1 to 3, in admixture with a pharmaceutically acceptable carrier or diluent.

14.     A pharmaceutical composition as claimed in claim 13, formulated for parenteral administration.

15.     A pharmaceutical composition as claimed in claim
13, formulated for oral administration.

Claims for AUSTRIA 0060659

CLAIMS

1.      A process for preparing a glyoxal-cysteine adduct containing free radicals, which process comprises reacting glyoxal and cysteine in solution and exposing the reaction product to oxygen.

2.      A process as claimed in claim 1, wherein the solution is an aqueous solution.

3.      A process as claimed in claim 1 or claim 2, wherein the reaction mixture is exposed to oxygen throughout the reaction period.

4.      A process as claimed in claim 3, wherein the reaction mixture is exposed to oxygen by bubbling oxygen through it.

5.      A process as claimed in any one of the preceding claims, wherein the reaction is effected by mixing an aqueous solution of cysteine with an aqueous solution of glyoxal.

6.      A process as claimed in claim 5, wherein both said aqueous solutions are saturated with oxygen.

7.    A process as claimed in any one of the preceding claims, wherein the reaction is effected at a pH of from 6.5 to 7.5, to prepare a 1:1 glyoxal:cysteine adduct.

8.    A process as claimed in any one of claims 1 to 6, wherein the reaction is effected at a pH of from 3 to 6.5, to prepare a 1:2 glyoxal:cysteine adduct.

9.    A process as claimed in claim 8, wherein said pH is about 5.5.

10.    A process as claimed in any one of the preceding claims, wherein said adduct is separated from the reaction mixture as a precipitate and is subsequently formulated with a pharmaceutical carrier or diluent to prepare a pharmaceutical composition.

**European Patent Office**

**EUROPEAN SEARCH REPORT**

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | J. AM. CHEM.SOC., vol.84, May 20, 1962, Washington D.C. (US), M. BODANSZKY et al.:"Degradation of thiostrepton. The structure of thiostreptine", pages 2003-2004 <br> * page 2003, right-hand column, lines 24,25 * | 1,2 | C 07 C   47/127 <br> C 07 C 149/247 <br> A 61 K   31/195 <br> A 61 K   31/11 |
| A | NATURE, vol.192, November 4, 1961, Basingstone (GB), A.B.K. NJAA:"Effect of derivatives of L-cysteine on growth of leuconostoc mesenteroides (8042)", pages 463-464 <br> * page 463, columns 1,2 * | 1 | |
| D,A | TETRAHEDRON, vol.32, 1976, Pergamon Press, Oxford (GB), H. ESTERBAUER et al.:"The reaction of cysteine with alpha, beta-unsaturated aldehydes", pages 285-289 <br> * page 285:Introduction * | 1 | **TECHNICAL FIELDS SEARCHED (Int. Cl. 3)** <br><br> C 07 C   47/00 <br> C 07 C 149/00 <br> A 61 K   31/00 <br> C 07 D 277/00 |

The present search report has been drawn up for all claims

| Place of search | Date of completion of the search | Examiner |
|---|---|---|
| THE HAGUE | 02-06-1982 | PAUWELS G.R.A. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
&  : member of the same patent family, corresponding document

EPO Form 1503. 03.82